# EUROPEAN PATENT APPLICATION

(11) **EP 2 116 555 A1**
(43) Date of publication of application: **11.11.2009**
(21) Application number: 08075485.6
(22) Date of filing: 08.05.2008
(51) Int. Cl.: C07K 16/18, A61K 51/10

(54) **Use of a radioactively labelled molecule specifically binding to ED-B fibronectin in a method of treatment of Hodgkin lymphoma**

(71) Applicant: Bayer Schering Pharma Aktiengesellschaft, 13353 Berlin (DE)
(72) Inventor: The designation of the inventor has not yet been filed

(57) **Abstract**

The invention relates to the use of a radioactively labelled molecule specifically binding to ED-B fibronectin in a method of treatment for patients with Hodgkin lymphoma, in particular primary refractory Hodgkin lymphoma or recurrent Hodgkin lymphoma after chemotherapy, and for Hodgkin lymphoma patients scheduled for primary combined-modality therapy.

SEQUENCE LISTING

<110>  Bayer Schering Pharma Aktiengesellschaft

<120>  Use of a radioactively labelled molecule specifically binding to
        ED-B fibronectin in a method of treatment of Hodgkin lymphoma

<130>  53763AEP

<160>  12

<170>  PatentIn version 3.3

<210>  1
<211>  116
<212>  PRT
<213>  Artificial

<220>
<223>  Vh of L19

<400>  1

<210>  2
<211>  108
<212>  PRT
<213>  Artificial

<220>
<223>  Vl of L19

<400>  2

<210> 3
<211> 12
<212> PRT
<213> Artificial

<220>
<223> Linker between Vh and Vl

<400> 3

Gly Asp Gly Ser Ser Gly Gly Ser Gly Gly Ala Ser
1               5                   10

<210> 4
<211> 116
<212> PRT
<213> Artificial

<220>
<223> etaCH4 domain

<400> 4

Gly Pro Arg Ala Ala Pro Glu Val Tyr Ala Phe Ala Thr Pro Glu Trp
1               5                   10                  15

Pro Gly Ser Arg Asp Lys Arg Thr Leu Ala Cys Leu Ile Gln Asn Phe
            20                  25                  30

Met Pro Glu Asp Ile Ser Val Gln Trp Leu His Asn Glu Val Gln Leu
        35                  40                  45

Pro Asp Ala Arg His Ser Thr Thr Gln Pro Arg Lys Thr Lys Gly Ser
    50                  55                  60

Gly Phe Phe Val Phe Ser Arg Leu Glu Val Thr Arg Ala Glu Trp Glu
65                  70                  75                  80

Gln Lys Asp Glu Phe Ile Cys Arg Ala Val His Glu Ala Ala Ser Pro
                85                  90                  95

Ser Gln Thr Val Gln Arg Ala Val Ser Val Asn Pro Glu Ser Ser Arg
            100                 105                 110

Arg Gly Gly Cys
        115

<210>  5
<211>  5
<212>  PRT
<213>  Artificial

<220>
<223>  Linker (L19 and etaCH4)

<400>  5

Ser Gly Gly Ser Gly
1               5

<210>  6
<211>  5
<212>  PRT
<213>  Artificial

<220>
<223>  CDR1 Vh

<400>  6

Ser Phe Ser Met Ser
1               5

<210>  7
<211>  17
<212>  PRT
<213>  Artificial

<220>
<223>  CDR2 Vh

<400>  7

Ser Ile Ser Gly Ser Ser Gly Thr Thr Tyr Tyr Ala Asp Ser Val Lys
1               5                   10                  15

Gly

<210>  8
<211>  7
<212>  PRT
<213>  Artificial

<220>
<223>  CDR3 VH

<400>  8

Pro Phe Pro Tyr Phe Asp Tyr
1               5

<210>  9
<211>  12
<212>  PRT
<213>  Artificial

<220>
<223>  CDR1 Vl

<400>  9

Arg Ala Ser Gln Ser Val Ser Ser Ser Phe Leu Ala
1               5                   10

<210>  10
<211>  7
<212>  PRT
<213>  Artificial

<220>
<223>  CDR2 Vl

<400>  10

Tyr Ala Ser Ser Arg Ala Thr
1               5

<210>  11
<211>  9
<212>  PRT
<213>  Artificial

<220>
<223>  CDR3 Vl

<400>  11

Gln Gln Thr Gly Arg Ile Pro Pro Thr
1               5

<210>  12
<211>  357
<212>  PRT
<213>  Artificial

<220>
<223>  L19-SIP monomer

<400>  12

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Phe
            20                  25                  30

Ser Met Ser Trp Val Arg Gln Ala Pro Gly Lys Gly Leu Glu Trp Val
        35                  40                  45

Ser Ser Ile Ser Gly Ser Ser Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Glu Ile Val Leu Thr Gln Ser Pro Gly Thr Leu Ser Leu Ser Pro Gly
    130                 135                 140

Glu Arg Ala Thr Leu Ser Cys Arg Ala Ser Gln Ser Val Ser Ser Ser
145                 150                 155                 160

Phe Leu Ala Trp Tyr Gln Gln Lys Pro Gly Gln Ala Pro Arg Leu Leu
                165                 170                 175

Ile Tyr Tyr Ala Ser Ser Arg Ala Thr Gly Ile Pro Asp Arg Phe Ser
            180                 185                 190

Gly Ser Gly Ser Gly Thr Asp Phe Thr Leu Thr Ile Ser Arg Leu Glu
        195                 200                 205

Pro Glu Asp Phe Ala Val Tyr Tyr Cys Gln Gln Thr Gly Arg Ile Pro
    210                 215                 220

Pro Thr Phe Gly Gln Gly Thr Lys Val Glu Ile Lys Ser Gly Gly Ser
225                 230                 235                 240

Gly Gly Pro Arg Ala Ala Pro Glu Val Tyr Ala Phe Ala Thr Pro Glu
                245                 250                 255

Trp Pro Gly Ser Arg Asp Lys Arg Thr Leu Ala Cys Leu Ile Gln Asn
            260                 265                 270

Phe Met Pro Glu Asp Ile Ser Val Gln Trp Leu His Asn Glu Val Gln
        275                 280                 285

Leu Pro Asp Ala Arg His Ser Thr Thr Gln Pro Arg Lys Thr Lys Gly
    290                 295                 300

Ser Gly Phe Phe Val Phe Ser Arg Leu Glu Val Thr Arg Ala Glu Trp
305                 310                 315                 320

Glu Gln Lys Asp Glu Phe Ile Cys Arg Ala Val His Glu Ala Ala Ser
                325                 330                 335

Pro Ser Gln Thr Val Gln Arg Ala Val Ser Val Asn Pro Glu Ser Ser
            340                 345                 350

Arg Arg Gly Gly Cys
        355

## Description

The invention relates to the use of a radioactively labelled molecule specifically binding to ED-B fibronectin in a method of treatment of Hodgkin lymphoma, in particular primary refractory Hodgkin lymphoma or recurrent Hodgkin lymphoma after chemotherapy.

### Brief description of Hodgkin lymphoma (HL):

HL is a rare malignant lymphoid tumor with an annual incidence of 2-3 cases per 100'000 persons in the Western world that predominantly affects people in their 2^{nd} and 3^{rd} decade of life. HL represents about 1% of all de novo neoplasms occurring every year worldwide, and between 7500 to 8000 new cases of HL are diagnosed each year in the USA. Non-tender, painless lymphadenopathy typically in the cervical nodal chain, is the most common clinical presentation of the disease, occurring in about 75% of cases. The diagnosis of HL is based upon the identification of very few characteristic and causative giant cells (Reed-Sternberg and Hodgkin cells) intermingled between inflammatory cells in disease-affected lymphatic tissues (usually enlarged lymph nodes). According to the REAL classification and the WHO schema, based on morphologic, phenotypic, genotypic, and clinical findings, HL can be subdivided in two main types: lymphocyte predominant HL (4-5% of all cases) and classic HL (96% of cases), which can be further subdivided into nodular sclerosis, mixed cellularity, lymphocyte depletion, and lymphocyte-rich classic HL (Harris, et al, 1999). However, for classic HL, the histopathologic classification has no impact on treatment decisions (Pileri, et al, 2005; Diehl, et al, 2005; Ng, et al, 2007). In the vast majority of cases (98%), the malignant Reed-Stemberg and Hodgkin cells derive from germinal center B cells (Kuppers, et al, 1994). Very rarely, they derive from T cells (Muschen, et al, 2000). The reactive milieu which is sustained by autocrine or paracrine production of various cytokines can represent 99% or more of the cell population in affected lymphatic tissues. HL evolves, due to currently unknown etiological factors, in a germinal center of a single lymph node and then spreads by contiguity to adjacent lymph node chains.

### Current disease management:

An excisional biopsy of a suspicious lymph node should be performed for initial diagnosis. A staging procedure including CT scans (chest, abdomen, pelvis), a bone marrow biopsy, and analysis for systemic symptoms and adverse prognostic factors, is necessary to identify the extent of disease (according to Cotswolds modified Ann Arbor classification) and, subsequently, the most suitable therapy for an individual patient.

Irrespective of histopathology, classical HL patients can be grouped into 3 major subsets in terms of clinical risk factors and treatment outcome: early-stage favorable HL, early-stage unfavorable HL, and advanced-stage HL. For these patient groups standard primary treatments were defined. Various and less well defined therapy options are currently in use for treating primary refractory disease or recurrent disease after chemotherapy. Primary treatment of the majority of HL patients currently is a combined-modality therapy, composed of an initial multi-agent chemotherapy, followed by involved-field external beam radiation.

### Standard therapies:

For *early-stage favorable HL* treatment currently is changing. Until recently, extended-field irradiation (30-40 Gy, irradiation of involved and adjacent lymph node regions) was considered standard treatment. However, because of the high relapse rate (30-40%) after initial complete response (90-98%) and the fatal long-term effects (secondary cancers, cardiovascular and pulmonary problems), extended-field radiotherapy is being replaced by a combined-modality approach consisting of limited amounts of chemotherapy (2-4 treatment cycles) followed by involved-field irradiation (20-30 Gy; Ferme, et al, 2007).

To facilitate radiation field design, the lymphatic system is divided into lymph node regions, based on the Rye classification for staging (Lukes, et al, 1966). An involved-field encompasses not only the visibly (on CT scans) involved enlarged lymph nodes but also other lymph nodes within the same lymph node region. Accordingly, in a patient presenting with a single enlarged cervical lymph node for example, involved-field radiotherapy would include the entire ipsilateral cervical chain of lymph nodes and all supra-clavicular lymph nodes, because these nodes are considered to belong to one and the same lymph node region. Insofar, the involved field (lymph node region) currently is the minimum radiation field size used for radiotherapy in HL patients. It is unclear, however, whether it is possible to reduce the involved-field radiation dose to 20 Gy or less or to restrict the radiation field to the actually enlarged lymph nodes within a given lymph node region without compromising the overall clinical outcome. Furthermore, in a central prospective review on all diagnostic imaging of HL study patients (HL10, HL11) a panel of independent expert radiation oncologists identified difficulties in controlling disease extension and defining the involved-field irradiation volume. Involved-field coverage was found suboptimal in 40% of 2792 reviewed cases (Eich, et al, 2008).

For *early-stage unfavorable HL patients* a combined-modality treatment approach is considered standard. However, among other factors such as the optimal chemotherapy regimen, and the number of chemotherapy cycles, the irradiation field sizes and the dosage of radiation within these fields are subjects of intense debate. Anyway, the treatment outcome for this patient group has improved dramatically during the past decades, mainly resulting from the use of combined-modality therapy. Historically, radiation or chemotherapy alone was associated with a high relapse rate of approximately 50%. Four cycles of effective chemotherapy followed by 30 Gy of involved-field radiotherapy is the standard treatment for patients with unfavorable-prognosis early-stage HL. Currently, most clinical trials are exploring new combinations of more effective chemotherapy and further reduced radiation doses to determine optimal treatment with the aim of decreasing late morbidity and mortality while maintaining a high probability of freedom of first recurrence (Diehl, et al, 2005).

*Advanced-stage HL patients* were considered incurable up to the middle of the 20^{th} century. With the advent of multi-agent multi-cycle chemotherapy, complete remissions could be achieved in the majority of this high-risk patient population. Several reports, however, have indicated that patients treated with chemotherapy alone failed to achieve long-lasting responses or progressed, primarily in previously involved nodal sites (Fabian, et al, 1994). Several trials investigated the role of consolidation radiotherapy after primary chemotherapy with divergent results. In a meta-analysis of 14 studies involving more than 1700 patients, two study designs were compared: in the additional design irradiation was added to the same chemotherapy, and in the parallel design two cycle of chemotherapy in one treatment arm were substituted for radiation therapy in the other treatment arm. In the additional design, radiotherapy reduced the hazard ratio of relapse by about 40%. However, no survival benefit was detectable for any subset of patients analyzed. In the parallel design, there was no significant difference in disease-free survival, but overall survival was significantly higher among patients treated with chemotherapy alone, since there were more deaths in the combined-modality group due to causes other than HL, including leukemia (Loeffler, et al, 1998). In a prospective EORTC study, patients in complete remission after chemotherapy were randomized to receive no further treatment or involved-field radiotherapy (30 Gy). Patients in partial remission (33%) after 6 cycles of chemotherapy who received involved-field radiotherapy significantly benefited from the addition of radiotherapy, since they achieved similar relapse-free and overall survival rates as compared to patients who experienced a complete remission after 6 cycles of chemotherapy and were not treated with radiotherapy (Aleman, et al, 2007). Insofar, recommended standard treatment for advanced HL patients is 6-8 chemotherapy cycles plus involved-field radiotherapy (20-30 Gy) for residual tumor and/or bulky disease.

*Primary refractory HL or_relapsed HL after chemotherapy:* Patients who relapse after achieving an initial complete remission after chemotherapy can achieve a second complete remission with salvage therapy. Such salvage therapy includes radiotherapy for localized relapse in previously non-irradiated areas, conventional salvage chemotherapy, or high-dose chemotherapy (HDCT) with autologous blood stem cell therapy (ASCT; Josting, et al, 2000). Patients who relapse after radiation therapy alone achieve satisfactory results with conventional chemotherapy. However, treatment of recurrent disease after initial chemotherapy or combined chemo-radiotherapy is less well defined. Salvage radiotherapy, conventional chemotherapy, salvage chemotherapy, and HDCT with ASCT can be tried. The assumed most active treatment in this setting, HDCT with ASCT in a prospective study resulted in a freedom from treatment failure in 55% of the patients (Schmitz, et al, 2002).

*Treatment of elderly patients with HL:* Patients older than 60 years (approximately 10-15% of all patients) fare much worse as compared to younger patients. This patient population usually experiences a high rate of toxicity during treatment and a high frequency of early relapses, suggesting the need for less toxic but effective treatment modalities. Currently, there is no specific standard treatment available for elderly HL patients.

### Treatment outcome and long-term toxicity:

The progress in developing risk-adapted treatment modalities for HL over the last 50 years is nicely reflected in the tumor-free survival rate at 10 years of between 80% and 90% and the fact that these tumor survivors will have a life expectancy almost equivalent to age-matched healthy individuals.

Unfortunately, there was a high rate of late-occurring adverse events of the initial therapy (up the mid 90ties) affecting 25%-30% of patients after 10-20 years. Such long-term complications after curative therapy, in particular after upper diaphragmatic irradiation, include lung (e.g. fibrosis), heart (e.g. myocardial damage causing heart insufficiency), thyroid dysfunction, secondary cancers (acute myeloid leukemia, breast and lung cancer), and gonadal dysfunction (loss of libido, sexual dysfunction, sterility). Much of this toxicity is actually caused by external-beam field radiation, a rather unselective approach that also damages normal tissues, in particular when given after chemotherapy. This rate of complications counterbalanced the initial success rates for high numbers of tumor-free survivors and resulted in a late decline of the survival curves due to deaths that were consequences of the treatment and not caused by the primary disease. Insofar, the question arises of how much over-treatment and long-term toxicity should be allowed to justify such high success rates of primary HL therapies, in particular the combined-modality treatment approach (Diehl, et al, 2005).

Although modem combined-modality treatments with reduced radiation fields, reduced doses or dose intensities have decreased the long-term complication rate to some extent, only time can tell whether the current, sometimes still aggressive treatments (e.g. combined modality therapy now standard also for early-stage favorable HL and advanced-stage HL with partial response to chemotherapy or bulky disease) will be superior to the former therapeutic options in terms of efficacy and long-term toxicity. Clinical trials have been designed to explore reduced intensity combined-modality therapies, with a focus on decreasing the radiotherapy dose to the utmost minimum, which could be 20 Gy (results still pending).

### Current limitations in managing patients with HL:

Although HL can be cured in the majority of patients, areas of medical concern remain and need to be addressed:
1. Insufficient treatment outcome for patients with primary refractory HL or recurrent HL after multiple chemotherapy regimens.
2. Lack of radiotherapy options for HL patients who relapse in pre-irradiated lymph node regions (no further significant external-beam radiation possible due to damaging normal tissues in the radiation field volume).
3. Presumed over treatment, in particular in the context of primary curative combined-modality therapies (chemo-radiotherapy; for all HL subgroups).
4. Restriction of external-beam radiation to enlarged lymph nodes within a lymph node region (confocal radiation within the involved field) potentially puts patients at risk of achieving an adequate overall outcome.
5. Insufficient therapy (in particular combined-modality therapy) for older HL patients (>60 a; 15% of overall population) in terms of efficacy and, more importantly, toxicity.
6. External-beam radiation (EBR) of HL patients is technically difficult and requires special additional training. In particular, in a quality control review, involved-field coverage was suboptimal in 40% of HL study patients (Eich, et al, 2008).

In principle, similar limitations also hold true for other hematologic malignancies that express ED-B fibronectin (Sauer, et al, 2006) and are treated with combined-modality therapy, including localized aggressive lymphoma (e.g. stage I and II diffuse large B cell lymphoma), localized indolent lymphoma (e.g. stage I and II follicular lymphoma, small lymphocytic lymphoma), and rare non-Hodgkin lymphomas such as MALT-lymphoma, peripheral T cell lymphoma, anaplastic large cell lymphoma, and mantle cell lymphoma.

Accordingly, there is high medical need for a tumor-targeted and thus selective radiotherapy option, such as ¹³¹I-L19-SIP radioimmunotherapy, that could be combined with external-beam radiotherapy (EBR), chemotherapy or be applied alone to further improve the overall treatment outcome of HL and potentially other lymphoma patients.

### ED-B fibronectin:

One of the most selective oncofetal markers associated with neo-angiogenesis and tissue remodeling known so far represents the extra domain B (ED-B) of Fibronectin (FN) (Castellani, et al, 1994). FNs are high molecular-weight extracellular matrix (ECM) components abundantly expressed in a range of healthy tissues and body fluids. Various different FN isoforms can be generated due to alternative splicing at the level of the primary transcript. The ED-B, a small domain of 91 amino acids, which is identical in sequence in mouse and man, is usually absent in both plasma and tissue-fibronectin, except for some blood vessels of the regenerating endometrium and the ovaries. (Alessi, et al, 2004). Increased binding affinity and valence of recombinant antibody fragments lead to improved targeting of tumoral angiogenesis (Viti, et al, 1999). However, it may become inserted in the fibronectin molecule during active tissue remodeling associated with neo-angiogenesis, thereby accumulating around the neo-vasculature and in the stroma of malignant tumors and in other tissues undergoing remodeling and angiogenesis. Recently, a number of good quality antibodies specific for the ED-B domain of fibronectin have been generated. In particular, the human single chain Fv antibody fragment scFv(L19), which displays a picomolar binding affinity for ED-B, has been verified to selectively target tumor neovasculature, both in experimental tumor models (Viti, et al, 1999) and in patients with cancer (Santimaria, et al, 2003). It was recently shown that considerable but variable vessel-associated ED-B expression was detected in various hematologic malignancies including Hodgkin's lymphoma (Sauer, et al., Poster at ASH conference, 2006).

The ED-B domain of fibronectin, a sequence of 91 amino acids identical in mice, rats and humans, which is inserted by alternative splicing into the fibronectin molecule, specifically accumulates around neovascular structures and represents a target for molecular intervention (Zardi, et al. 1987; Camemolla, et al. 1989; Castellani, et al, 1994). Using the human recombinant antibody L19 directed to the ED-B domain, the possibility of in vivo neovasculature targeting has been demonstrated in different tumour models (Tarli, et al. 1999; Viti, et al. 1999).

Monoclonal antibodies specifically recognising the ED-B - fibronectin domain are described in WO 97/45544.

Monoclonal antibody L19 is described in WO 99/58570.

WO 01 / 62298 on page 8, line 12 refers to the L19 VH and L19 VL domain sequences described in Pini et al. (1998) J. Biol. Chem. 273: 21769-21776. Pini, et al. describes parts of the sequence of L19 in Table II on page 21772. L19 has the EMBL accession Number AJ 006113.

The principles of radiotargeting for cancer therapy are described by Kassis AI (2005) in Expert Opin. Drug Delivery Vol.: 2(6), 981-991 and by Press OW, et al (2000) in Seminars in Oncology Vol.:27, No.6 (Suppl. 12), 62-73.

L19-SIP and its use for radioimmunotherapy are described in WO 03/076469. Berndorff, et al (2005) Clin. Cancer Res. Vol.:11 (19 Suppl.), 7053S-7063S described the use of ¹³¹1 -L19-SIP as preferred antibody format for radioimmunotherapy of solid tumours by targeting the extra domain B fibronectin. "SIP" stands for small immunoprotein. L19-SIP is an antibody format wherein the L 19-scFv is linked to an εs2-CH4 domain of IgE, and wherein two monomeric chains form a homodimer covalently linked by an S-S bridge (see e.g. WO03/076469; Borsi, et al., 2002). CH4 is the domain that allows dimerization in the IgE molecule and the εs2- isoform contains a cysteine at the carboxyterminal end, which stabilizes the IgE-dimer through an inter-chain disulphide bond. In the final SIP molecule of L19-SIP the ScFv(L19) is connected to the εₛ₂CH4 domain by a GGSG linker. The disclosure of WO03/076469 is included by reference.

There is a strong medical need for a medicament to effectively treat Hodgkin lymphoma, in particular primary refractory HL or recurrent HL after chemotherapy. There is also a strong medical need for reducing the external-beam radiotherapy dose or the radiotherapy field or both in HL patients of all subgroups eligible for combined-modality therapy in order to reduce the long-term toxicity rate of this treatment.

The present invention relates to the use of a radioactively labelled molecule specifically binding to ED-B fibronectin in a method of treatment of Hodgkin lymphoma patients.

In a preferred embodiment, the invention relates to the use of a radioactively labelled molecule specifically binding to ED-B fibronectin in a method of treatment of primary refractory HL or recurrent HL after chemotherapy.

The invention also relates to method of treatment of Hodgkin lymphoma patients, in particular primary refractory HL or recurrent HL after chemotherapy, comprising administering a therapeutically effective amount of a radioactively labelled molecule specifically binding to ED-B fibronectin.

In particular, the Hodgkin lymphoma is selected from classical Hodgkin lymphoma (cHL), especially nodular sclerosis, mixed cellularity, lymphocytic depletion, or lymphocyte-rich classic Hodgkin lymphoma. However, the embodiment also applies to lymphocyte-predominant Hodgkin lymphoma (HL), but treatment modalities differ strikingly as compared to patients with classic HL. In particular, combined-modality therapy is rarely used in lymphocyte-predominant Hodgkin lymphoma which tends to take a benign course after resection.

In a particularly preferred embodiment, for the patients suffering from primary refractory HL or recurrent HL after chemotherapy, no further effective treatment is available. These patients are considered suffering from end-stage HL.

Preferably, the treatments and uses of the present invention result in prolonged progression-free survival of a patient.

It was surprisingly found that the treatment with ¹³¹I-L19-SIP was highly effective in terms of inducing a near-complete remission (¹⁸F-FDG-PET scan and CT scan) and at the same time was surprisingly well tolerated in an advanced patient with end-stage classic HL. Therefore, the radioactively labelled molecules are surprisingly suitable for monotherapy of HL, in particular for patients with primary refractory HL or recurrent HL after chemotherapy.

Therefore, in a preferred embodiment, the radioactively labelled molecules can surprisingly be used for monotherapy. The molecules may be administered as a single treatment or as repeated treatments, e.g. full dose every 4-6 weeks. Due to the favourably short serum PK profile (approximately 24 hours) of ¹³¹I-L19-SIP and its long tumor-selective residential time (at least 96 hours), other treatment schedules (e.g. daily, every other day, weekly, or biweekly) may also be possible, depending on delivered radioactivity dose and overall acute toxicity.

The dosage of the radioactively labelled molecules, in particular radiolabelled L19-SIP, administered will vary according to the mode of use and route of use, as well as to the requirements of the patient. In general, a single dosage in man is in the range of about 2 - 200 MBq/kg body weight (10 - 1,000 µg/kg body weight). The radiolabelled antibody must be given in a dose that does not cause toxicity to the most radiosensitive organ in the body (dose limiting organ). In animal experiments the red bone marrow was identified as dose limiting organ for ¹³¹I -labelled antibodies.

Primary refractory HL or recurrent HL patients after chemotherapy typically are in a bad clinical shape and frequently suffer from severe adverse effects of previous treatments as well as from cancer progression. Nevertheless, these patients are sometimes treated with high doses of involved-field radiation to multiple sites using external-beam radiotherapy (EBR). As the EBR subsequent to chemotherapy results in severe adverse events and late toxicity, radiotherapy regimes which are better tolerated due to a decreased radiation exposure are needed. Similarly, combined-modality therapy as a primary or sometimes 2^{nd} line therapy for HL patients of all risk groups, is associated with a relatively high rate of late toxicity, mostly due to the radiation part (radiation dose, size of radiation filed) of the combined-modality therapy. Insofar, radiotherapy regimens which are better tolerated in terms of late toxicity, but are at least equally effective are needed also for the primary combined-modality therapy of HL patients (including all risk groups).

The invention also relates to a method of treatment of Hodgkin lymphoma, comprising administering a therapeutically effective amount of at least one radioactively labelled molecule specifically binding to ED-B fibronectin, followed by or accompanied by fractionated EBR at standard or reduced radiotherapy dose.

The invention also relates to the use of a radioactively labelled molecule specifically binding to ED-B fibronectin in a method of treatment of Hodgkin lymphoma, preferably primary refractory Hodgkin lymphoma or recurrent Hodgkin lymphoma patients after chemotherapy comprising administering a therapeutically effective amount of at least one radioactively labelled molecule specifically binding to ED-B fibronectin, wherein the administration of the molecule is followed by or accompanied by fractionated EBR at standard or reduced radiotherapy dose.

Therefore, in another preferred embodiment, radioactively labelled molecules may be administered in combination with EBR such that the radioactively labelled molecule, in particular ¹³¹I-L19-SIP, is applied, followed by or accompanied by fractionated EBR at standard, e.g. for refractory disease, or reduced, e.g. for primary therapy, radiotherapy dose over a certain time frame. Such time frame is typically between 1 and 50 days, preferably between 7 and 21 days, depending on overall applied dose. The application time of ¹³¹I-L19-SIP is typically between 10 minutes and several hours, in particular between about 30 minutes and 5 hours. In particular, the application time is about 60 minutes. The administration is preferably performed intravenously.

The invention specifically relates to a method of treatment of HL patients, in particular primary refractory HL or recurrent HL patients after chemotherapy and HL patients of all clinical risk subtypes scheduled for primary combined-modality therapy, comprising
a) administering a therapeutically effective amount of a radioactively labelled molecule specifically binding to ED-B fibronectin, and
b) delivering EBR exclusively to the involved field

EBR is preferably individually adapted to a reduced dose according to dosimetry after ¹³¹I-L19-SIP application for primary therapy in order to reduce long-term toxicity or standard dose for relapsed/refractory patients in order to overcome refractory disease.

Steps a) and b) may be performed simultaneously or successively.

The involved field is preferably understood as the diseased lymph node region.

More preferably, the radioactively labelled antibody is administered first, followed by EBR over 1-4 week time frame.

Therefore, in a further preferred embodiment, radioactively labelled molecules may be administered to HL patients scheduled for primary combined-modality therapy, wherein EBR is given simultaneously or after administration of the radioactively labelled molecule, in particular ¹³¹I-L19-SIP, wherein the EBR dose is reduced compared to a standard EBR dose at least by about 20%, preferably at least by about 30%, most preferred at least by about 50%.

The invention also relates to a method of treatment of HL patients, in particular elderly HL patients scheduled for primary combined-modality therapy, comprising
a) administering a therapeutically effective amount of a radioactively labelled molecule specifically binding to ED-B fibronectin, and
b) delivering EBR exclusively to the involved field
such that the EBR dose in step a) is reduced compared to a standard EBR dose at least by about 20%, preferably at least by about 30 %, most preferred at least by about 50%.

"Standard EBR" is understood as a radiation dose of about between 20 and 30 Gy being delivered in a fractionated fashion of about 2 Gy daily over a time frame of up to about 4 -6 weeks.

In another preferred embodiment, radioactively labelled molecules may be administered to HL patients scheduled for primary combined-modality therapy, including all clinical risk subgroups, or for primary refractory HL or recurrent HL patients after chemotherapy scheduled for radiotherapy or chemoradiotherapy, wherein EBR given simultaneously or after administration of the radioactively labelled molecule, in particular ¹³¹I-L19-SIP, is strictly restricted to the initially enlarged lymph nodes. Even more preferably, EBR is applied in a confocal fashion to strikingly reduce the size of the EBR radiation field. The EBR dose may be standard (20-30 Gy) or be reduced at least by about 20%, preferably at least by about 30%, most preferred at least by about 50%, depending on the individual clinical situation. Preferably, the EBR dose is reduced. Both proposed modifications to the current EBR schedules are intended to substantially reduce long-term and/or late toxicity of combined chemoradiotherapy for HL patients, without compromising on anti-tumor efficacy.

Surprisingly excellent localization of ¹³¹I-L19-SIP selective to HL lesions and more surprisingly, excellent treatment response associated with negligible toxicity in this patient, were observed.

Thus, the methods of treatments and uses of the present invention provide for prolonged progression-free and overall survival and/or reduced long-term and/or late toxicity of combined-modality (chemoradiotherapy) treatment for Hodgkin lymphoma, such as secondary malignancies, cardiovascular and pulmonary problems and hormonal/gonadal deficiencies.

Therefore, the present invention also relates to the use of a radioactively labelled molecule specifically binding to ED-B fibronectin in a method of reducing long-term and/or late toxicity of combined-modality (chemoradiotherapy) treatment of Hodgkin lymphoma patients. In a preferred embodiment, the long-term and/or late toxicity is selected from secondary malignancies, cardiovascular problems and psychomotoric deficiencies.

Therefore, the present invention also relates to use of a radioactively labelled molecule specifically binding to ED-B fibronectin in a method of achieving prolonged progression-free survival in HL patients.

The invention also relates to a method of reducing long-term and/or late toxicity of combined chemoradiotherapy treatment of Hodgkin lymphoma patients, in particular selected from secondary malignancies, cardiovascular and pulmonary problems and hormonal deficiencies, comprising administering a therapeutically effective amount of a radioactively labelled molecule specifically binding to ED-B fibronectin.

In yet another preferred embodiment, the radioactively labelled molecules of the invention are used for treating high risk Hodgkin lymphoma, in particular selected from extensive disease, bulky disease and slow responding Hodgkin lymphoma patients. In particular, the radioactively labelled molecules of the invention are administered after standard chemotherapy, e.g. COPP/ABVD or BEACOPP regimen, followed by EBR. This treatment surprisingly leads to an enhanced chance of complete remission (CR).

The invention therefore relates to the use of at least one radioactively labelled molecule specifically binding to ED-B fibronectin in a method of treating Hodgkin lymphoma patients, in particular Hodgkin lymphoma patients scheduled for combined-modality therapy, wherein the administration of the molecule is preceded by standard chemotherapy and is followed by EBR.

Due to the extremely low toxicity, treatment with the radioactively labelled molecule specifically binding to ED-B fibronectin can even be performed simultaneously with chemotherapy, for example COPP/ABVD or BEACOPP regimen.

The invention therefore relates to the use of at least one radioactively labelled molecule specifically binding to ED-B fibronectin in a method of treating Hodgkin lymphoma patients, in particular Hodgkin lymphoma patients scheduled for combined-modality therapy, wherein chemotherapy, for example COPP/ABVD or BEACOPP regimen, is performed simultaneously.

The invention also relates to a method of achieving prolonged progression-free survival and/or for reducing long-term and/or late toxicity of combined chemoradiotherapy treatment of Hodgkin lymphoma patients, in particular selected from secondary malignancies, cardiovascular and pulmonary problems and hormonal deficiencies, comprising administering a therapeutically effective amount of a radioactively labelled molecule specifically binding to ED-B fibronectin simultaneously to chemotherapy, e.g. with the COPP/ABVD or BEACOPP regimens.

The invention also relates to a method of treatment of patients with HL, in particular those scheduled for combined-modality therapy, comprising following steps:
a) performing standard chemotherapy,
b) administering a therapeutically effective amount of at least one radioactively labelled molecule specifically binding to ED-B fibronectin, and
c) delivering EBR

The EBR dose may be standard dose or reduced dose or reduced radiation field, e.g. exclusively limited to the affected lymph nodes or involved tissues.

The patient is preferably a human.

Various administration routes are possible, e.g. intravenous, subcutaneous or intraperitoneal administration, wherein the intravenous administration is preferred.

Therapeutic formulations of the active agents used in accordance with the present invention are prepared for storage by mixing an active agent having the desired degree of purity with optional pharmaceutically acceptable carriers, excipients or stabilizers (Osol, 1980), in the form of lyophilized formulations or aqueous solutions.

In a preferred embodiment, the molecule specifically recognizing ED-B fibronectin is an antibody or antibody mimetic, in particular an antibody.

Preferably, the antibody is human.

In a preferred embodiment, the antibody specifically binds to the ED-B-domain of fibronectin (FN). Such antibodies are known in the prior art and are e.g. described in WO 97/45544.

In another embodiment, the antibody specifically recognizing ED-B-fibronectin binds to a cryptic epitope. An example for such antibody is the BC-1 antibody.

Preferably, such antibody which binds to the ED-B domain of fibronectin exhibits a high affinity for the ED-B-domain of FN, in particular, the antibody binds to the ED-B fibronectin domain with nanomolar or subnanomolar affinity. Such antibodies are known in the prior art and are e.g. described in WO99/58570. In particular preferred is the L19 antibody.

The antibody specifically recognizing ED-B fibronectin, in particular the L19 antibody, can be employed in various antibody formats. Preferred antibody formats are full IgG, Fab, (Fab')₂, scFv, diabody, minibody or small immunoprotein (SIP) format. Especially preferred are the full IgG, scFv and SIP format for the L19 antibody. Most preferred is the L19 antibody in the scFv format. Several immunoprotein formats are known in the prior art, e.g. based on the CH3 domain or the εₛ₂-CH4 domain of IgE. The preferred SIP format for L 19 based on the εₛ₂-CH4 domain of IgE and L 19 in full IgG format are for example described in WO03/076469.

In a further preferred embodiment, the antibody contains at least one CDR sequence of the L 19 antibody.

In an especially preferred embodiment, the antibody comprises the CDR sequences of the L19 antibody; in particular it comprises the sequences according to SEQ ID no. 6 to 11.

In a further preferred embodiment, the antibody comprises the VL and VH chain of the L19 antibody. In a preferred embodiment, it comprises least one VH chain according to SEQ ID No. 01 or at least one VL chain according to SEQ ID No. 02. In an especially preferred embodiment, it comprises least one VH chain according to SEQ ID No. 01 and at least one VL chain according to SEQ ID No. 02.

In a further preferred embodiment, the antibody comprises one VH chain according to SEQ ID No. 01 and one VL chain according to SEQ ID No. 02. In a further preferred embodiment, the antibody comprises two VH chains according to SEQ ID No. 01 and two VL chains according to SEQ ID No. 02.

In a further preferred embodiment, the VH and the VL chains are connected by an antibody linker.

In a preferred embodiment, the antibody linker comprises a sequence according to SEQ ID No. 03, or a sequence having at least 90% identity to the sequence according to SEQ. ID. No. 03.

In a further preferred embodiment, the antibody is present in dimeric form. Such antibody may be in a small immunoprotein format (SIP), in particular based on the CH3 or on the εₛ₂CH4 domain, particularly preferred on the εₛ₂CH4, most preferred on the εₛ₂CH4 domain according to SEQ ID No. 4.

In a particularly preferred embodiment, L19-SIP in its monomeric form comprises the sequence according to SEQ ID No. 12, most preferred L19-SIP has the sequence according to SEQ ID No. 12.

Preferably, the antibody is human.

The antibody may be monomeric, or multimeric, e.g. dimeric. Dimeric or other multimeric forms may be formed covalently or non-covalently. L19(scFv) and AP39 may be used in monomeric form. L19-SIP is preferably used in dimeric form.

In particular, for L19-SIP, the monomers are linked by a S-S bridge, as described in Borsi, et al., Int. J. Cancer, 2002, 102: 534-539. Alternatively, the L19 antibody may be used in full IgG format. In a preferred embodiment, L19-SIP forms covalent dimers.

In another preferred embodiment, the antibody is in monomeric form. In particular, the L19-antibody is in scFv format, as described in WO99/58570 or in scFv format with tags as described in WO 03/055917. In particular AP38 or AP39 described in WO 03/055917 can be used according to the invention.

The antibodies are preferably produced recombinantly using methods known to the skilled person. In particular, prokaryotic or eukaryotic expression systems, e.g. yeast or mammalian expression systems, can be used.

The molecules specifically binding to ED-B fibronectin are radioactively labelled.

Methods for labelling antibodies of the invention are disclosed in Berndorff, et al., Clin. Cancer Res., 2005; 11 (Suppl.), p. 7053s-7063s.

Preferably, the radioisotope is suitable for therapeutic application, in particular therapeutic application in humans.

In a preferred embodiment, the radioisotope is a radioisotope of an element selected from Re, In, Y, Lu, or I or a mixture thereof.

In an even more preferred embodiment, the radioisotope is selected from ¹²⁴I,¹²⁵I, ¹³¹I,, ¹⁸⁶Re, ¹⁸⁸Re ²⁰³Pb, ⁶⁷Ga, ⁴³Sc, ⁴⁷Sc, ¹¹¹In, ⁹⁷R,u ⁶⁷Cu, ⁹⁰Y, ¹²¹Sn, ¹⁵³Sm, ¹⁶⁶Ho, ¹⁰⁵Rh, ¹⁷⁷Lu, ³²P, ³³P, ⁵⁹Fe, ⁷⁷As, ⁸⁹Sr, ¹⁰⁹Pd,¹¹¹Ag, ^{117m}Sn, ¹⁴²Pr,¹⁶¹Tb, ¹⁶⁶Dy, ¹⁶⁹Er, ¹⁹⁴Ir, ¹⁹⁸Au, ¹⁹⁹Au, ²¹²Pb, ²²⁵Ac, ²¹¹At, ²¹²Bi, ²¹³Bi, ²²³Ra, ²²⁴Ra and/or ¹⁷²Lu or a mixture thereof.

Most preferred is the use of ¹³¹I and ⁹⁰Y.

Many appropriate radiotherapeutic agents are known in the art, as are methods for their attachment to e.g. antibodies (see e.g., US 5,021,236 and US 4,472,509). Certain attachment methods involve the use of a metal chelate complex employing, for example, an organic chelating agent such a DTPA attached to the antibody (US 4,472,509). Radioactively labelled antibodies can also be iodinated by contact with sodium or potassium iodide and a chemical oxidising agent such as sodium hypochlorite (Redshaw, et al, 1974, or an enzymatic oxidizing agent, such as lactoperoxidase (Marchalonis, et al, 1969). Radioactive labelling with ¹³¹I is preferably performed directly, by covalent linkage to the antibody.

In an especially preferred embodiment of the present invention, an ¹³¹I-labelled antibody is used, wherein the antibody in its monomeric form comprises the CDR sequences of L19.

In an even more preferred embodiment, the ¹³¹I-labelled antibody in its monomeric form comprises a Vl domain of L19, a Vh domain of L19 and an εₛ₂-CH4 domain.

In a particularly preferred embodiment, an ¹³¹I-labeled antibody selected from L19(scFv), AP39, AP38, and L19-SIP is used.

Most preferred is the use of ¹³¹I labelled L19-SIP (¹³¹I-L19-SIP).

Antibody linker is any linker, preferably a peptide linker, which is suitable for linking Vh and Vl domains. Suitable linkers are for example described in Bird, et al, 1988; Huston, et al, PNAS USA, 85, 5879-5883, 1988; EP 0 573 551; EP 0 623679 and EP 0 318554, which documents are introduced by reference.

"Specifically binding" or "specifically recognizing" as used herein refers to binding to the corresponding target. Typically, the binding molecule, antibody, antibody fragment or antibody mimetic binds with an affinity of at least about 1x10⁻⁷ M, preferably of at least about 1x10⁻⁹ M, and binds to the predetermined target with an affinity that is at least two-fold greater than its affinity for binding to a non-specific target (e.g. BSA, casein) other than the predetermined target or a closely-related target.

"Antibody" as used herein encompasses full length antibodies, comprising native antibodies, monoclonal antibodies, polyclonal antibodies and multispecific antibodies (e.g., bispecific antibodies), human antibodies, humanized antibodies, chimeric antibodies, and full IgG antibodies, as well as antibody fragments.

The term "antibody fragment" refers to a portion of a full length antibody, in which a variable region or a functional capability is retained, namely the specific binding to the target. Examples of antibody fragments include, but are not limited to, a Fab, Fab', F(ab')2, Fd, Fv, scFv and scFv-Fc fragment, a diabody, a linear antibody, small immunoprotein formats, a single-chain antibody, a minibody, a diabody formed from antibody fragments, and multispecific antibodies formed from antibody fragments. Antibody fragments are usually smaller than full antibodies. Thereby, the pharmacokinetics are different and some antibody fragments only consist of one polypeptide chain, which can make production easier. Preferably, the antibody fragment is in scFv, (scFv)2, or small immunoprotein format. The small immunoprotein format can be a format based on a CH3-domain (for example described in US 5,837,821) or εₛ₂CH4-domain of human IgE (for example described in WO 03/076469).

The term "monoclonal antibody" (mAb) refers to an antibody obtained from a population of substantially homogeneous antibodies; that is, the individual antibodies comprising the population that are identical except for naturally occurring mutations that may be present in minor amounts. Monoclonal antibodies are highly specific, being directed against a single antigenic determinant, also referred to as an epitope. The modifier "monoclonal" is indicative of a substantially homogeneous population of antibodies directed to the identical epitope and is not to be construed as requiring production of the antibody by any particular method. Monoclonal antibodies can be made by any technique or methodology known in the art; for example, the hybridoma method first described by Koehler, et al., 1975, Nature 256:495, or recombinant DNA methods known in the art (see, e.g., U.S. Patent No. 4,816,567). In another example, monoclonal antibodies can also be isolated from phage antibody libraries, using techniques described in Clackson, et al., 1991, Nature 352: 624-628, and Marks, et al., 1991, J. Mol. Biol. 222: 581-597.

In contrast, the antibodies in a preparation of polyclonal antibodies are typically a heterogeneous population of immunoglobulin isotypes and/or classes and also exhibit a variety of epitope specificity.

The term "chimeric" antibody as used herein is a type of monoclonal antibody in which a portion of or the complete amino acid sequence in one or more regions or domains of the heavy and/or light chain is identical with, homologous to, or a variant of the corresponding sequence in a monoclonal antibody from another species or belonging to another immunoglobulin class or isotype, or from a consensus sequence.

Certain types of antibody fragments can be generated by enzymatic treatment of a full-length antibody. Papain digestion of antibodies produces two identical antigen-binding fragments called "Fab" fragments, each with a single antigen-binding site, and a residual "Fc" fragment, so called because of its ability to crystallize readily. The Fab fragment also contains the constant domain of the light chain and the CH1 domain of the heavy chain. Pepsin treatment yields a F(ab')2 fragment that has two antigen-binding sites and is still capable of cross-linking antigen.

Fab' fragments differ from Fab fragments by the presence of a few additional residues at the C-terminus of the CH1 domain, including one or more cysteines from the antibody hinge region. Fab-SH is the designation herein for a Fab' in which the cysteine residue(s) of the constant domains bear a free thiol group. F(ab')2 antibody fragments are pairs of Fab' fragments linked by cysteine residues in the hinge region. Other chemical couplings of antibody fragments are also known.

"Fv" is a minimum antibody fragment that contains a complete antigen-recognition and binding site consisting of a dimer of one heavy and one light chain variable domain in tight, non-covalent association. In this configuration, the three CDRs of each variable domain interact to define an antigen-binding site on the surface of the VH VL dimer. Collectively, the six CDRs confer antigen-binding specificity to the antibody.

A "single-chain Fv" or "scFv" antibody fragment is a single chain Fv variant comprising the VH and VL domains of an antibody, in which the domains are present in a single polypeptide chain and which is capable of recognizing and binding antigen. The scFv polypeptide optionally contains a polypeptide linker positioned between the VH and VL domains that enables the scFv to form a desired three-dimensional structure for antigen binding (see, e.g., Pluckthun, 1994, In The Pharmacology of Monoclonal Antibodies, Vol. 113, Rosenburg and Moore eds., Springer-Verlag, New York, pp. 269-315).

The term "diabodies" refers to small antibody fragments having two antigen-binding sites. Each fragment contains a heavy chain variable domain (VH) concatenated to a light chain variable domain (VL). By using a linker that is too short to allow pairing between the two domains on the same chain, the linked VH-VL domains are forced to pair with complementary domains of another chain, creating two antigen-binding sites.

Diabodies are described more fully, for example, in EP 404,097; WO 93/11161; and Hollinger, et al., 1993, Proc. Nat. Acad. Sc. USA 90: 6444-6448.

A humanized antibody or a humanized antibody fragment includes an immunoglobulin amino acid sequence variant, or fragment thereof, which is capable of binding to a predetermined antigen and which, comprises one or more framework regions (FRs) having substantially the amino acid sequence of a human immunoglobulin and one or more CDRs having substantially the amino acid sequence of a non-human immunoglobulin. This non-human amino acid sequence is referred to herein as an "import" sequence, which is typically taken from an "import" antibody domain, particularly a variable domain. In general, a humanized antibody includes at least the CDRs or HVLs of a non-human antibody, inserted between the FRs of a human heavy or light chain variable domain.

"Native antibodies" are defined herein as heterotetrameric glycoproteins, typically of about 150,000 daltons, composed of two identical light (L) chains and two identical heavy (H) chains. Each light chain is covalently linked to a heavy chain by one disulfide bond to form a heterodimer. The heterotetramer is formed by covalent disulfide linkage between the two identical heavy chains of such heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (VH), followed by three or four constant domains (CH1, CH2, CH3, and CH4), as well as a hinge region between CH1 and CH2. Each light chain has two domains, an amino-terminal variable domain (VL) and a carboxy-terminal constant domain (CL). The VL domain associates non-covalently with the VH domain, whereas the CL domain is commonly covalently linked to the CH1 domain via a disulfide bond. Particular amino acid residues are believed to form an interface between the light and heavy chain variable domains (Chothia, et al., 1985). The term "hypervariable" refers to the fact that certain sequences within the variable domains differ extensively in sequence among antibodies and contain residues that are directly involved in the binding and specificity of each particular antibody for its specific antigenic determinant. Hypervariability, both in the light chain and the heavy chain variable domains, is concentrated in three segments known as complementarity determining regions (CDRs) or hypervariable loops (HVLs). CDRs are defined by sequence comparison in Kabat, et al., 1991, In: Sequences of Proteins of Immunological Interest, 5th Ed. Public Health Service, National Institutes of Health, Bethesda, MD., whereas HVLs are structurally defined according to the three-dimensional structure of the variable domain, as described by Chothia and Lesk, 1987, J. Mol. Biol. 196: 901-917.

Where these two methods result in slightly different identifications of a CDR, the structural definition is preferred. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain.

The term "label" refers to a detectable compound or composition that is conjugated directly or indirectly to the molecule binding to ED-B fibronectin, in particular to the antibody.

Although fibronectins (FNs) are the product of the single FN gene, the resulting protein can exist in multiple forms which - apart from posttranslational modifications - arise from alternative splicing of its primary RNA transcript. This polymorphism which leads to as many as 20 different isoforms in human FN, thereby generating FNs with different solubility, cell adhesive and ligand-binding properties, provides cells with the possibility to modify the composition of the extracellular matrix (ECM) in a tissue-specific manner. Alternative splicing takes place in three regions of the primary RNA transcript: Exon usage or skipping leads to either inclusion or omission of two type-III repeats, extra-domain B (ED-B, also termed EIIIB or EDIII), which is inserted between FN type-III repeats III7 and IIIB, or/and extra-domain A (EDA, also termed EIIIA or EDI), inserted between FN type-III repeats III11 and III12. This type of splicing occurs in many vertebrates, including Xenopus, chicken, rat, dog and human. "ED-B" domain" is to be understood as the extra-domain B of human fibronectin. It is often referred to as ED-B, EIIIB or EDII.

"Antibody mimetics" are understood as binding molecules based on protein frameworks ("scaffolds") which specifically bind to the target and which are distinct from antibodies and antibody fragments. Such scaffolds are described in Binz, et al., 2005, Nat. Biotechnol. 23, 1257-1268. Antibody mimetics specifically binding to ED-B fibronectin are described in Grabulovski, et al., J. Biol. Chem., 2007, 282:3196-3204.

ε_{S2}-CH4 is defined in Erqiu Li, et al. "Mammalian cell expression of dimeric small immuno proteins (SIP)" (1997) Protein Engineering Vol.: 10, no. 6 pages 731 - 736. The sequence of the domain is shown in SEQ ID No. 4.
SEQ ID No. 1 represents the Vh chain of the L19-SIP antibody.
SEQ ID No. 2 represents the Vl chain of the L19-SIP antibody.
SEQ ID No. 3 represents the antibody linker linking Vl and Vh the L19-SIP antibody.
SEQ ID No. 4 represents the εₛ₂-CH4 domain part of L19-SIP
SEQ ID No. 5 represents the linker linking the scFv part of L19-SIP and the εₛ₂-CH4 domain.
SEQ ID No. 6 to 11 represents the CDR sequences of the L19 antibody.
SEQ ID No. 12 represents the sequence of L19-SIP in its monomeric form.

### Figure Legend:

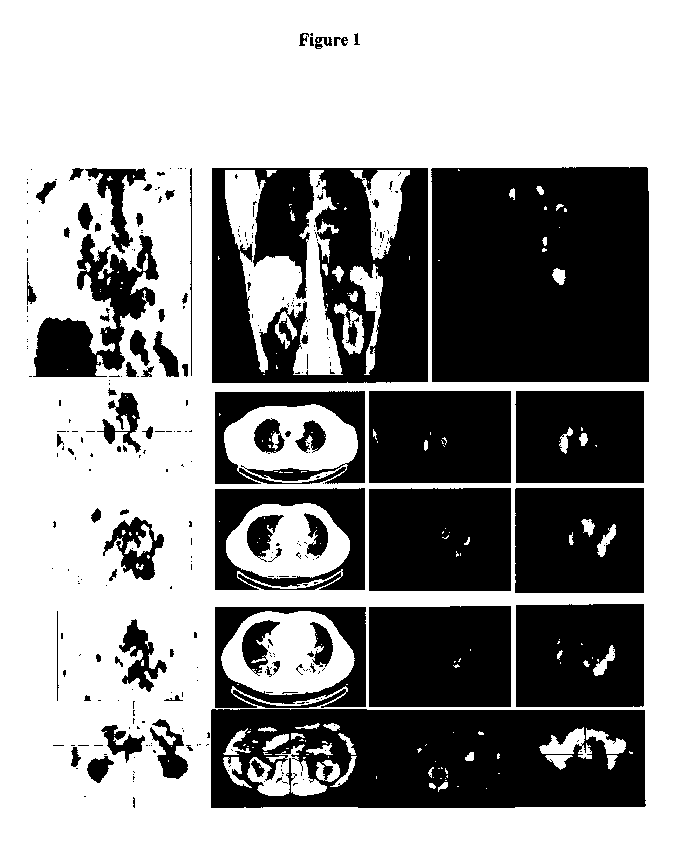

Figure 1: ¹³¹I-L19-SIP uptake in lymphoma lesions in a patient with Hodgkin lymphoma:
   ¹⁸F-FDG PET scans show intense glucose metabolism in multiple enlarged mediastinal lymph-nodes, intrapulmonary lesions (left-most column, first four images; intrapulmonary lesion marked), as well as in lumbo-aortic lymph nodes (left-most image in lowest row), indicating clinically active lymphoma lesions. This patient also received intravenous injections of 185 MBq and, subsequently in therapeutic intent, 5.55 GBq of ¹³¹I-L19-SIP. Whole-body and SPECT-CT images were obtained at day 12 post-administration of this dose to confirm specific tumor targeting. SPECT-CT coronal (upper right panel) and transaxial images of the thorax (rows 2-4) as well as the upper-abdomen (lowest row), are shown, demonstrating selective uptake of ¹³¹I-L19-SIP into the multiple ¹⁸F-FDG avid lymphomatous lesions.

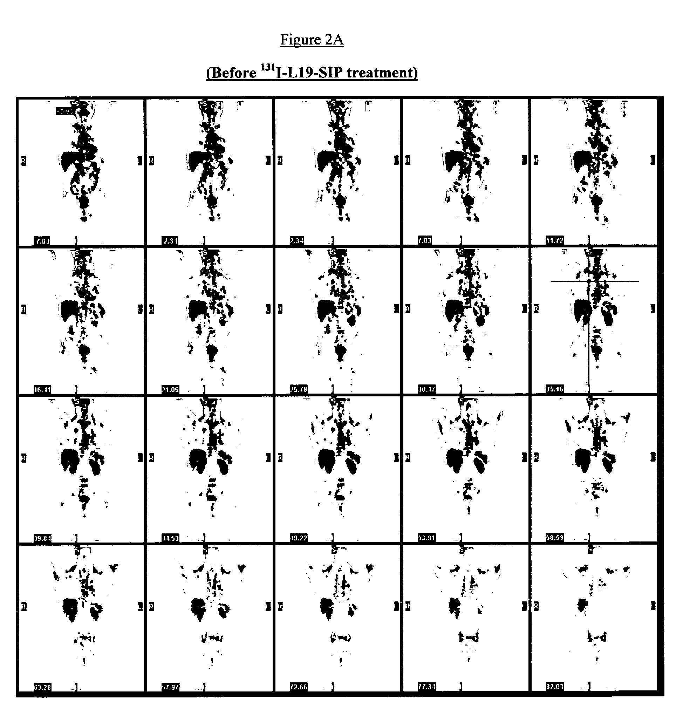

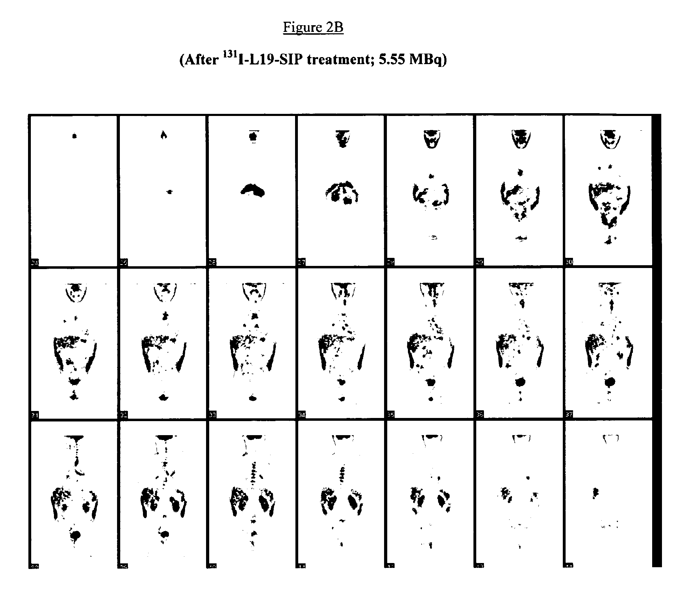

Figure 2: ¹⁸F-FDG PET scans in a Hodgkin lymphoma patient before and after radioimmunotherapy with ¹³¹I-L19-SIP:
   Based on selective uptake into tumor lesions and on adequate bone marrow dosimetry (lesion/red-marrow absorbed dose ratio ≥10), this Hodgkin lymphoma patient received a therapeutic dose of ¹³¹I-L19-SIP (5.55 GBq; 150 mCi), subsequent to the diagnostic dose. ¹⁸F-FDG PET scans were performed prior (Fig 2a) and 4 weeks after (Fig 2b) radioimmunotherapy, showing an almost complete disappearance of ¹⁸F-FDG uptake to the multiple lymph node enlargements after treatment, including the previously external beam irradiated lumbo-aortic lymph nodes. This finding is strongly predictive for the induction of a clinically meaningful tumor response in Hodgkin lymphoma patients (Gallamini, et al 2007 and 2008). The absorbed radioactivity dose to the intrapulmonary target lesion (largest pulmonary lesion, see Fig 1) was dosimetrically estimated to be approximately 14 Gy, whereas the respective red bone marrow dose was estimated to be approximately 1.3 Gy. At 4 weeks after radioimmunotherapy with ¹³¹I-L19-SIP, classical CT scans disclosed that the patient had achieved an excellent partial response according to RECIST criteria (Table 1).

### References:

Aleman BM, Raemaekers JM, Tomisic R, et al. Involved-field radiotherapy for patients in partial remission after chemotherapy for advanced Hodgkin's lymphoma. Int J Radiat Oncol Biol Phys 67:19-30, 2007.
Alessi P, Ebbinghaus C, Neri D., Viti F, Tarli L, Giovannoni L, Zardi L, Neri D. Molecular targeting of angiogenesis. Biochim Biophys Acta 1654:39-49, 2004.
Berndorff, et al., Radioimmunotherapy of solid tumors by targeting extra domain B fibronectin: identification of the best-suited radioimmunoconjugate. Clin. Cancer Res. 11 (Suppl.):7053s-7063s, 2005
Bird, et al, Science 242, 423-426, 1988.
Bombardieri E, Coliva A, Chiesa C, et al. Phase I study with antifibronectin I-131-L19SIP: First dosimetric and therapeutic results. J Nuclear Med 48 suppl 2. abs 1681 pp. 398p,2007
Borsi L, Balza E, Bestagno M, et al., Selective targeting of tumoral vasculature: comparison of different formats of an antibody (L19) to the ED-B domain of fibronectin. Int J Cancer 102:75-85, 2002.
Camemolla B, Balza E. Siri A, et al. A tumor-associated fibronectin isoform generated by alternative splicing of messenger RNA precursors. J Cell Biol 108 :1139 - 1148, 1989.
Castellani P, Viala G, Dorcaratto A, et al, The fibronectin isoform containing the ED-B oncofetal domain: a marker of angiogenesis. Int J Cancer 59:612-618, 1994.
Castellani P, Viale G, Dorcaratto A, Nicolo G, Kaczmarek J, Querze G, Zardi L. The fibronectin isoform containing the ED-B oncofetal domain: a marker of angiogenesis. Int J Cancer 59:612-8, 1994. Erratum in: Int J Cancer 62:118, 1995.
Chothia, et al., 1985, J Mol. Biol. 186:651-663
Chothia and Lesk, 1987, J. Mol. Biol. 196: 901-917
Clackson, et al., 1991, Nature 352: 624-628,
Diehl V, Re D, Josting A. Hodgkin lymphoma: clinical manifestations, staging, and therapy. Chapter 75, pp 1347-1377. In Hematology, basic principles and practice, 4th edition, ed. R. Hoffman, EJ Benz, S. Shattil, B. Furie, H. Cohen, L. Silberstein, P. McGlave. 2005 Elsvier Inc, Philadelphia PA.
Eich HT, Engenhart-Cabillic R, Hansemann K, et al. Quality control of involved field radiotherapy in patients with early-favorable (HD10) and early-unfavorable (HD11) Hodgkin's lymphoma: an analysis of the German Hodgkin Study Group. Int J Radiat Oncol Biol Phys. 2008 EPUB
Fabian C, Mansfield C, Dahlberg S, et al. Low-dose involved field radiation after chemotherapy in advanced Hodgkin lymphoma: A Southwest Oncology Group randomized study. Ann Intern Med 120:903-912, 1994.
Ferme C, Eghbali H, Meerwaldt JH, et al. Chemotherapy plus involved-field radiation in early-stage Hodgkin lymphoma. N Engl J Med 357:1986-71, 2007
Gallamini A, Hutchings M, Rigacci L, et al. Early interim 2(18F(fluoro-2-deoxy-d-glucose positron emission tomography is prognostically superior to international prognostic score in advanced-stage Hodgkin's lymphoma: a report from a joint Italian-Danish study. J Clin Onc 25:3746-3752, 2007.
Gallamini A, Hutchings M, Ariqdor A, Polliack A. Early interim PET scan in Hodgkin lymphoma: where do we stand? (Review) Leuk Lymphoma 49:659-62, 2008.
Harris NL, Jaffe ES, Diebold J, et al. World Health Organization classification of neoplastic disease of the hematopoietic and lymphoid tissues: report of the clinical advi sory committee meeting - Airlie House, Virginia, November 1997. J Clin Oncol 17:3835-3849, 1999.
Hollinger, et al., 1993, Proc. Nat. Acad. Sc. USA 90: 6444-6448
Huston, et al, PNAS USA. 85, 5879-5883, 1988.
Josting A, Wolf J, Diehl V. Hodgkin lymphoma: prognostic factors and treatment strategies. Curr Opin Oncol 12:403-411, 2000.
Kabat, et al., 1991, In: Sequences of Proteins of Immunological Interest, 5th Ed. Public Health Service, National Institutes of Health, Bethesda, MD.
Kassis AI in Expert Opin. Drug Delivery 2:981-99, 2005.
Koehler, et al., 1975, Nature 256:495
Kuppers R, Rajewski K, Zhao M, et al. Hodgkin lymphoma: Hodgkin and Reed-Stemberg cells picked from histological sections show clonal immunoglobuline rearrangements and appear to be derived from B cells at various stages of development. Proc Natl Acad Sci U S A 91:10962-10966, 1994.
Loeffler M, Brosteanu O, Hasenclever D, et al. Meta-analysis of chemotherapy versus combined modality treatment trials in Hodgkin's disease. International Database on Hodgkin's disease. Overview Study Group. J Clin Onc 16:818-829, 1998.
Lukes RJ, Carver LF, Hall TC, et al. Report of the nomenclature committee in symposium: Obstacles to the control of Hodgkin lymphoma. Cancer Res 26:1311, 1966.
Marchalonis J.J., Biochem. J., 113, 299, 1969
Marks, et al., J Mol Biol 222:581-597, 1991
Muschen M, Rajewski K, Brauninger A, et al. Rare occurrence of classical Hodgkin lymphoma as a T cell lymphoma. J Exp Med 191:387-394, 2000.
Ng A, Weiss L, Fredman A. Hodgkin lymphoma. Chapter 73; pp 1689-1711. In Clinical Radiation Oncology, 2nd edition, 2007; eds Gunderson and Tepper, Elsevier, Churchill Livingston, Philadelphia, PA.
Osol A. Ed.Remington's Pharmaceutical Sciences 16th edition, 1980.
Pileri SA, Falini B, Stein H. Pathobiology of Hodgkin's lymphoma. Chapter 74, pp 1325-1346. In Hematology, basic principles and practice, 4th edition, ed. R. Hoffman, EJ Benz, S. Shattil, B. Furie, H. Cohen, L. Silberstein, P. McGlave. 2005 Elsvier Inc, Philadelphia, PA.
Pini A, Viti F, Satuccia A, et al. Design and use of a phage display library. Human antibodies with subnanomolar affinity against a marker of angiogenesis eluted from a two-dimensional gel. J Biol Chem 273: 21769-21776, 1998
Pluckthun, 1994, In The Pharmacology of Monoclonal Antibodies, Vol. 113, Rosenburg and Moore eds., Springer-Verlag, New York, pp. 269-315
Press OW, et al. Seminars in Oncology 27 (Suppl. 12):62-73, 2000.
Redshaw M.R., Lynch S.S., J. Endocrinol., 60, 527, 1974.
Santimaria M, Moscatelli G, Viale GL, Giovannoni L, Neri G, Viti F, Leprini A, Borsi L, Castellani P, Zardi L, Neri D, Riva P. Immunoscintigraphic detection of the ED-B domain of fibronectin, a marker of angiogenesis, in patients with cancer. Clin Cancer Res 9:571-579, 2003.
Sauer, et al., Poster at ASH conference, 2006
Schmitz N, Pfistner B, Sextro M, et al. Aggressive conventional chemotherapy compared with high-dose chemotherapy with autologous haematopoietic stem-cell transplantation for relapsed chemosensitive Hodgkin's disease. A randomized trial. Lancet 359:2065-2071, 2002.
Tarli, et al. (1999) Blood, Vol.: 94, pages 192 - 198
Viti, et al. (1999) Cancer Res. Vol.: 347
Viti F, Tarli L, Giovannoni L, Zardi L, Neri D. Increased binding affinity and valence of recombinant antibody fragments lead to improved targeting of tumoral angiogenesis. Cancer Res 1999;59:347-352.
Zardi, et al. (1987) EMBO J. Vol.: 6, pages 2337 - 2342

### Example

¹³¹I-L19-SIP was intravenously injected in a 28-year-old male patient with nodular sclerosis Hodgkin lymphoma. This patient had failed multiple prior chemotherapies, external-beam radiation therapy to the lumbo-aortic lymph node enlargements (36 Gy total), and immunotherapy. He presented with relapsed disease as documented on CT and ¹⁸F-FDG PET-CT scans. One-hundred-eighty-five MBq (5 mCi) of ¹³¹I-L19-SIP were first administered to this patient for biodistribution and dosimetry evaluations. Since an excellent ratio of ¹³¹I-L19-SIP uptake to lymphoma nodes as compared to red bone marrow was observed, the patient subsequently was treated with a therapeutic dose of ¹³¹I-L19-SIP (5.5 GBq; 150 mCi). This treatment was clinically uneventful and was associated only with a mild, uncomplicated, and transient thrombocytopenia that spontaneously resolved. Whole-body, spot and SPECT-CT images (GE Infinia™ Hawkeye®) were acquired at different time-points after ¹³¹I-L19-SIP injection, as described (Bombardieri, et al, 2007). In particular, ¹³¹I-L19-SIP SPECT-CT images shown here were acquired 12 days post-injection of the radioimmunotherapy dose. Dosimetry to normal organs and to lymphoma lesions were estimated as absorbed dose (in Gy) after the application of the diagnostic dose of ¹³¹I-L19-SIP (185 MBq).

It was surprisingly found that at 4 weeks after radioimmunotherapy with ¹³¹I-L19-SIP, classical CT scans disclosed that the patient had achieved an excellent partial response according to RECIST criteria (Table 1).

It was also surprisingly found in ¹⁸F-FDG PET scans that were performed prior (Fig 2a) and 4 weeks after (Fig 2b) radioimmunotherapy, that there is an almost complete disappearance of ¹⁸F-FDG uptake to the multiple lymph node enlargements after treatment, including the previously external beam irradiated lumbo-aortic lymph nodes.

**Table 1. CT-documented lymphoma lesion in a Hodgkin lymphoma patient before and after radioimmunotherapy with ¹³¹I-L19-SIP**

| | Pre-Treatment (mm) | 4 weeks Post-Treatment (mm) |
|---|---|---|
| Superior lobe, right lung | 9.5 x 8 | 7.5 6.4 |
| Superior lobe, left lung | 11.3 x 7 | 7.8 x 3 |
| Retro-aortic lymph node | 28 x 15 | 14 x 7.6 |
| Retro-caval lymph node | 18 | 12 |

## Claims

1. Use of a radioactively labelled molecule specifically binding to ED-B fibronectin in a method of treatment of Hodgkin lymphoma patients, preferably selected from patients suffering from primary refractory Hodgkin lymphoma or recurrent Hodgkin lymphoma patients after chemotherapy.

2. Use of a radioactively labelled molecule specifically binding to ED-B fibronectin in a method of achieving prolonged progression-free or overall survival and/or of reducing long-term and/or late toxicity of combined chemoradiotherapy treatment of Hodgkin lymphoma patients, wherein the radioactively labelled molecule specifically binding to ED-B fibronectin is administered simultaneously with chemotherapy.

3. Use according to claim 1, wherein the treatment is a monotherapy.

4. Use according to any of claims 1 to 2 wherein, in addition, EBR is given exclusively to the involved field of a patient simultaneously or successively with the application of a radioactively labelled molecule specifically binding to ED-B fibronectin.

5. Use according to claim 4, wherein the EBR dose is reduced compared to a standard EBR dose at least by about 20%, preferably at least by about 30 %, most preferred at least by about 50%.

6. Use according to any of claims 1 to 5, wherein the Hodgkin lymphoma patient is an elderly patient.

7. Use according to any of claims 4 to 6, wherein the EBR field is restricted in size as compared to a standard EBR field to those lymph nodes only in a given lymph node region, that were affected at presentation, wherein radiation preferably is performed in a confocal fashion and radiation dose is standard, or reduced at least by about 20 %.

8. Use according to any of claims 1, 2 and 4 to 7, wherein the administration of the molecule is preceded by standard chemotherapy and is followed by EBR.

9. Use of a radioactively labelled molecule specifically binding to ED-B fibronectin in a method of treatment of Hodgkin lymphoma, preferably primary refractory Hodgkin lymphoma or recurrent Hodgkin lymphoma patients after chemotherapy comprising administering a therapeutically effective amount of at least one radioactively labelled molecule specifically binding to ED-B fibronectin, wherein the administration of the molecule is followed by or accompanied by fractionated EBR at standard or reduced radiotherapy dose.

10. Use of a radioactively labelled molecule specifically binding to ED-B fibronectin in a method of reducing long-term and/or late toxicity of combined-modality therapy (chemoradiotherapy) of Hodgkin lymphoma patients, in particular selected from secondary malignancies, cardiovascular and pulmonary problems and hormonal and gonadal deficiencies.

11. Use of at least one radioactively labelled molecule specifically binding to ED-B fibronectin in a method of treating Hodgkin lymphoma patients, wherein chemotherapy is performed simultaneously.

12. Use according to any of claims 1 to 11, wherein the molecule specifically binding to ED-B fibronectin is an antibody or antibody mimetic.

13. Use according to claim 12, wherein the antibody specifically binds to the ED-B-domain of fibronectin.

14. Use according to claim 12 or 13, wherein the antibody comprises the CDR sequences of the L19 antibody.

15. Use according to any of claims 12 to 14, wherein the antibody is in full IgG, Fab, (Fab')2, scFv, diabody, minibody or small immunoprotein (SIP) format.

16. Use according to claim 14 or 15, wherein the antibody is selected from L19(scFv), AP38, and AP39.

17. Use according to any of claims 15 to 16, wherein the antibody in small immunoprotein (SIP) format comprises the εₛ₂CH4 domain.

18. Use according to claim 17, wherein the antibody is L19-SIP.

19. Use according to any of claims 1 to 18, wherein the molecule specifically binding to ED-B fibronectin is radioactively labelled with a radioactive isotope selected from Re, In, Y, Lu, or I or a mixture thereof.

20. Use according to claim 19, wherein the molecule specifically binding to ED-B fibronectin is radioactively labelled with ¹³¹I or ⁹⁰Y.

21. Use according to any of claims 1 to 20, wherein the radioactively labelled molecule specifically binding to ED-B fibronectin is ¹³¹I-L19-SIP.
